# FASCICULE DE BREVET EUROPEEN

(11) **EP 1 396 243 B1**
(45) Date de publication et mention de la délivrance du brevet: **15.08.2007**
(21) Numéro de dépôt: 02019937.8
(22) Date de dépôt: 04.09.2002
(51) Int. Cl.: A61F 5/00

(54) **Anneau chirurgical pourvu d'un système de commande à distance et réversible de la variation de son diamètre**
Chirurgischer Ring mit Fernsteuerungseinrichtung für reversible Durchmesserveränderungen
Surgical ring with remote control system for reversible variation of diameter

(43) Date de publication de la demande: 10.03.2004
(73) Titulaire: EndoArt S.A., Ecublens (VD) (CH)
(72) Inventeur: Bachmann, Michel André, 1126 Vaux sur Morges (CH)
(74) Mandataire: Vossius & Partner

(56) Documents cités:
- WO-A-00/15158
- WO-A-01/49245
- FR-A- 2 797 181
- US-A- 4 118 805

## Description

La présente invention se rapporte au domaine technique des implants chirurgicaux destinés à être implantés dans le corps d'un patient autour d'organes biologiques constituant une poche ou un conduit, et plus particulièrement aux anneaux gastriques conçus pour traiter l'obésité par implantation d'un anneau gastrique souple, destiné à former une boucle fermée autour de l'estomac pour réduire le diamètre de l'ouverture du stoma, l'invention se rapportant plus particulièrement aux anneaux de gastroplastie à commande à distance, n'impliquant aucune opération invasive postérieurement à sa mise en place.

La présente invention concerne un anneau chirurgical destiné à être implanté dans le corps d'un patient autour d'organes biologiques constituant une poche ou un conduit pour, d'une part former une boucle fermée entre ses deux extrémités formant ainsi respectivement une première et une deuxième extrémité et, d'autre part, réduire le diamètre de l'ouverture dudit organe lorsqu'il est enserré par l'anneau, ledit anneau comprenant un système pour commander de manière précise et réversible la variation du diamètre de l'anneau.

L'invention concerne plus particulièrement un anneau de gastroplastie, mais elle peut aussi concerner un anneau conçu pour être utilisé pour traiter l'incontinence urinaire ou fécale (sphincter artificiel) ou encore un anneau conçu pour régler le débit sanguin dans des vaisseaux sanguins par exemple, cette liste n'étant nullement limitative.

La présente invention concerne plus particulièrement un anneau de gastroplastie à commande à distance évitant ainsi toute ré-intervention chirurgicale invasive de réglage du diamètre de l'anneau, ledit anneau étant alors intégré dans un système de restriction et de contrôle à distance de l'ingestion d'aliments dans l'estomac d'un patient, comportant un anneau gastrique pourvu d'un actionneur relié à une antenne de réception pour recevoir un signal de commande, ainsi qu'une antenne émettrice disposée à l'extérieur du patient pour envoyer un signal de commande à l'antenne de réception, puis à l'actionneur.

Dans son application préférentielle, l'anneau conforme à l'invention est commandé par radiofréquence, l'actionneur étant d'une part, relié à l'antenne de réception par un circuit de réception dans lequel est intégré ladite antenne et d'autre part, comportant un boîtier électrique de commande relié à l'antenne émettrice.

Il est déjà connu d'intervenir de manière chirurgicale sur des patient atteints d'obésité extrêmement sévère (obésité morbide), c'est-à-dire dans le cas de patient dont le poids excède par exemple le poids idéal d'au moins 50 kg en implantant des anneaux de gastroplastie sur de tels patients. De telles interventions permettent d'éviter non seulement une série de problèmes de santé graves provenant d'un tel surpoids, mais encore et surtout d'éviter une mort certaine et proche de ces patients.

Il est en effet acquis que les patients souffrant d'obésité morbide voient leur espérance de vie réduite de manière importante, et d'au moins une dizaine à une quinzaine d'années tout en créant d'importants problèmes de charge psychologique. Par ailleurs, toute une série de phénomènes annexes de santé sont impliqués, ayant une incidence sur l'apparition de maladies annexes telles que des maladies cardio-vasculaires ou encore telles que l'hypertension, le diabète ou encore des arthrites sévères notamment.

Il est également acquis que, pour de tels patients, les traitements basés sur des diètes sévères combinées à une série d'exercices physiques, associés également à une modification du comportement, notamment alimentaire, sont peu adaptés, même si ces méthodes de traitement sont reconnues comme étant les plus saines.

C'est la raison pour laquelle les traitements efficaces et à long terme de l'obésité morbide font intervenir un traitement chirurgical.

De manière générale, on distingue les techniques de traitement chirurgical faisant intervenir un défaut d'absorption des aliments, c'est-à-dire un raccourcissement du passage des aliments et des sucs digestifs et les techniques faisant intervenir une restriction gastrique réduisant la taille de l'estomac.

Les techniques chirurgicales impliquant un défaut d'absorption sont celles impliquant par exemple une technique de « *by pass* » ou de dérivation du petit intestin, ou encore celles mettant en oeuvre une séparation du passage des aliments relativement aux sucs digestifs. Ces techniques chirurgicales sont relativement lourdes et peuvent donner lieu à de sévères complications, et c'est la raison pour laquelle elles ne sont plus guère utilisées maintenant.

On tend en effet maintenant à utiliser des techniques chirurgicales qui mettent en oeuvre des interventions chirurgicales plus réduites, telle que la restriction gastrique impliquant la pose d'un anneau gastrique.

Ces techniques sont maintenant d'utilisation assez courante et pour la plupart mettent en oeuvre, tel que décrit par exemple dans le brevet US-5 074 868, une bande souple en matériau élastomère destinée à être implantée autour de l'estomac, en formant une boucle fermée définissant un diamètre fixe pré-établi de l'anneau grâce à un système de fermeture. Le corps de la bande souple comporte une cavité ou chambre de compression à volume variable, qui est reliée à un cathéter de réglage permettant d'injecter ou de retirer un fluide dans la chambre de compression, de manière à faire varier le diamètre interne de la boucle pour modifier ou régler le diamètre du stoma. Ainsi, en combinaison avec le diamètre fixe et pré-établi de l'anneau, on peut régler dans une faible proportion le diamètre de l'anneau, ce qui permet de réguler le diamètre du stoma et donc de régler la quantité d'aliments ingérés.

Ces dispositifs connus donnent généralement satisfaction mais souffrent néanmoins d'un certain nombre d'inconvénients liés essentiellement aux difficultés provenant des interventions chirurgicales effectuées après la pose de l'anneau gastrique. En effet, il s'avère qu'en dépit de la possibilité de pouvoir modifier dans une certaine mesure le diamètre de l'anneau sans intervention chirurgicale majeure, grâce à la présence d'un boîtier miniaturisé implanté sous la peau du patient, la pose de tels anneaux peut s'accompagner de phénomènes d'intolérance, accompagnés par exemple de vomissements, liés à diverses causes et notamment à une trop forte réduction du diamètre du stoma, ou encore à une action inefficace de l'anneau liée à un diamètre du stoma trop important, ou encore tout simple à un gêne ou une infection ou une inflammation locale ou générale.

C'est la raison pour laquelle il s'avère souvent nécessaire d'intervenir à nouveau de manière chirurgicale, soit pour soulager le patient, soit pour modifier ou changer l'anneau préalablement implanté. De telles interventions chirurgicales sont particulièrement sévères et nécessitent le plus souvent la découpe de l'anneau par un chirurgien, s'accompagnant de son changement et de son remplacement.

En définitive, de telles opérations sont difficiles à réaliser, difficilement supportées par le patient, coûteuses et ce d'autant plus qu'elles impliquent la destruction d'un anneau et son remplacement.

Avec ces techniques classiques, on constate également une perte progressive et graduelle de pression à l'intérieur de l'anneau due à un phénomène naturel d'osmose à travers la paroi ce qui nécessite des interventions régulières pour réajuster le diamètre de l'anneau et contribue à accroître le besoin de surveillance constante du patient.

Enfin, il s'avère que le recours à une simple seringue généralement remplie d'eau physiologique pour assurer le réajustement diamétral de l'anneau par injection du liquide à travers un boîtier sous-cutané relié à l'anneau est une opération qui par sa simplicité peut échapper à tout contrôle médical et être effectuée par le patient lui-même. Les conditions optimales de sécurité et de contrôle ne sont donc pas remplies dans le cas de ces techniques classiques.

Pour tenter de remédier à ces inconvénients, il a déjà été proposé, tel que décrit par exemple dans la demande de brevet EP-0 876 808 de réaliser un anneau gastrique qui soit réglable d'une manière non invasive et sans inconfort pour le patient en commandant à distance, sans intervention chirurgicale invasive, des moyens électromagnétiques implantés dans le corps du patient et montés au moins en partie sur l'anneau gastrique. Les moyens mis en oeuvre comprennent une boîte de commande implantée dans l'anneau et raccordée à un réservoir de fluide, également implanté dans le corps du patient et chargé, à l'aide d'une pompe commandée de l'extérieur du corps du patient par l'intermédiaire de moyens électromagnétiques, d'injecter ou retirer du fluide dans l'anneau gastrique afin de régler son diamètre. L'ensemble du dispositif est sous la commande d'un moyen de contrôle externe, du genre micro-ordinateur équipé d'un émetteur-récepteur radio par exemple sous contrôle du médecin traitant.

Un tel dispositif marque bien évidemment une évolution intéressante et bénéfique pour les patients, mais souffre néanmoins d'un certain nombre d'inconvénients liés en particulier à la nécessité d'implanter dans le corps même du patient un réservoir de fluide, dont l'implantation est délicate et dont l'étanchéité est difficile à réaliser, ce qui peut représenter un danger pour le patient. Par ailleurs, un tel dispositif nécessite une source d'énergie interne, par exemple une pile, implantée dans le corps même du patient, ce qui une nouvelle fois complique l'intervention chirurgicale et surtout confère une certaine fragilité générale au système, et peut nécessiter une intervention chirurgicale destinée à changer la pile.

Le document WO-01/49245 A décrit un anneau chirurgical correspondant au préambule de la revendication 1.

L'invention vise en conséquence à porter remède aux différents inconvénients énumérés précédemment, et à proposer un nouvel anneau chirurgical présentant un système de commande réversible de la variation de son diamètre, qui soit particulièrement simple, sûr et efficace, notamment en matière de précision et de rendement, de telle sorte qu'il puisse être intégré dans un système de commande à distance ne nécessitant pas une énergie importante de commande.

Un autre objet de l'invention vise à proposer un nouvel anneau chirurgical, notamment gastrique dont la commande mécanique soit particulièrement précise.

Un autre objet de l'invention vise à proposer un nouvel anneau chirurgical, notamment gastrique, susceptible de présenter une forme circulaire en utilisation, tout en procurant une grande sécurité de commande.

Un autre objet de l'invention vise à proposer un nouvel anneau chirurgical, notamment gastrique permettant de mettre en oeuvre des moyens mécaniques particulièrement éprouvés et résistants, de manière à obtenir un anneau gastrique d'une grande robustesse et d'une bonne longévité.

Un autre objet de l'invention vise à proposer un nouvel anneau chirurgical, notamment gastrique qui, tout en permettant une bonne maîtrise de la variation du diamètre de l'anneau, est susceptible de minimiser les phénomènes d'intolérance par le patient.

Un autre objet de l'invention vise à proposer un nouvel anneau chirurgical, notamment gastrique dont le système de commande est particulièrement économe en matière d'énergie utilisée.

Un autre objet de l'invention vise à proposer un nouvel anneau gastrique d'encombrement particulièrement réduit, permettant une implantation facile dans l'estomac du patient.

Un autre objet de l'invention vise à proposer un nouvel anneau chirurgical, notamment gastrique qui permet une bonne répartition de l'ensemble des efforts de fermeture de l'anneau sur l'estomac.

Un autre objet de l'invention vise à proposer un nouveau système de restriction et de contrôle à distance de l'ingestion d'aliments dans l'estomac d'un patient qui soit particulièrement efficace, d'une grande robustesse et d'une bonne longévité, tout en requérant une énergie d'alimentation relativement faible.

Les objets assignés à l'invention sont atteints à l'aide des caractéristiques des revendications.

D'autres objets et avantages de l'invention apparaîtront mieux à la lecture de la description ci-jointe, ainsi qu'à l'aide des dessins annexés, à titre purement illustratif et informatif, dans lesquels :
- La figure 1 représente, selon une vue en coupe longitudinale, un exemple d'une première variante de réalisation d'un anneau gastrique conforme à l'invention.
- La figure 2 représente une vue de la section transversale de l'anneau gastrique représenté à la figure 1, ladite coupe étant effectuée selon la ligne II-II de la figure 1.
- La figure 3 représente, selon une vue en coupe longitudinale, une seconde variante de réalisation d'un anneau gastrique conforme à l'invention.
- La figure 4 représente la section transversale de l'anneau gastrique illustré à la figure 3, ladite coupe étant effectuée selon la ligne IV-IV de la figure 3.
- La figure 5 illustre, selon une vue en perspective et selon une vue en coupe longitudinale partielle, un exemple de réalisation d'un anneau gastrique conforme à l'invention et correspondant à la seconde variante de réalisation illustrée à la figure 3, ledit anneau étant équipé d'une antenne de réception en position dépliée.
- La figure 6 représente, selon une vue en perspective, un anneau gastrique identique à celui illustré à la figure 5, l'antenne de réception étant en position dépliée de fonctionnement.
- La figure 7 illustre, selon une vue en perspective, un anneau gastrique conforme à l'invention illustrant la sortie des fils de connexion électrique.
- La figure 8 illustre, selon une vue en coupe transversale, la position repliée du circuit de réception de l'anneau avec l'antenne de réception.
- La figure 9 illustre, selon une vue en coupe transversale, le circuit de réception avec son antenne en position dépliée.
- La figure 10 illustre, selon une vue en perspective partielle, un détail de réalisation de l'élément filiforme souple conforme à l'invention et servant au réglage du diamètre de l'anneau.

Dans la description qui suit il sera fait référence, uniquement à titre d'exemple, à un anneau gastrique conçu pour être implanté autour de l'estomac pour réduire le diamètre de l'ouverture du stoma, ou autour de l'oesophage. En effet, l'invention n'est nullement limitée à cette application et vise au contraire à couvrir d'autres anneaux chirurgicaux, tels ceux utilisés pour traiter l'incontinence urinaire ou fécale ou ceux utilisés autour de vaisseaux sanguins pour régler le débit sanguin.

Dans le cas de traitement d'incontinence urinaire, l'anneau sera implanté autour de la vessie ou des voies urinaires, et dans le cas d'incontinence fécale, il sera implanté autour des voies gastro-intestinales, et notamment autour des structures anales de l'intestin.

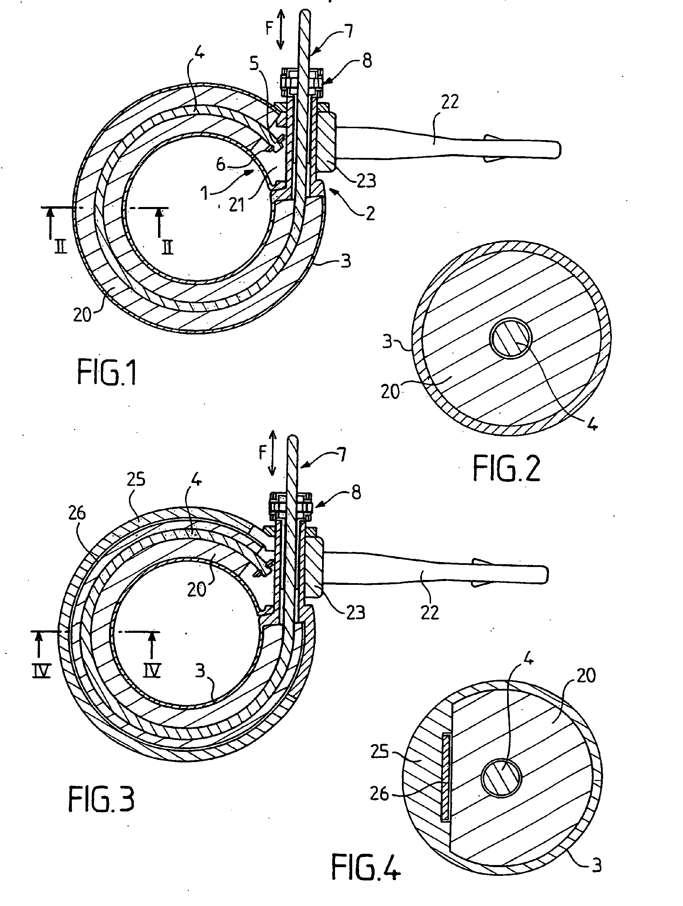

Les figures 1 à 10 illustrent un anneau gastrique conforme à l'invention destiné à être implanté autour de l'estomac d'un patient en formant une boucle sensiblement circulaire, afin de réaliser une restriction gastrique en réduisant le diamètre de l'ouverture du stoma. L'anneau gastrique conforme à l'invention se présente sous la forme d'une bande flexible tubulaire, dont l'enveloppe souple et élastique présente une surface lisse pour la rendre atraumatique de manière à être facilement supportée par le patient et les tissus de l'estomac. La bande est réalisée par exemple en matériau élastomère.

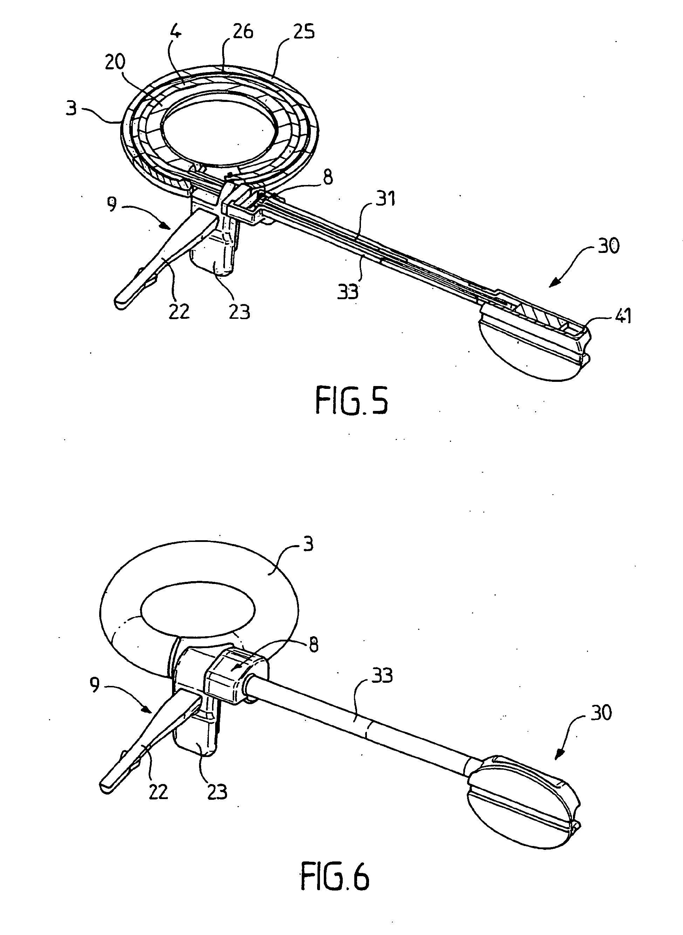

La bande flexible tubulaire comporte deux extrémités, respectivement 1, 2, sur lesquelles sont conformés et implantés des moyens de fermeture 9 (figures 5 et 6) destinés à coopérer de manière à assurer le verrouillage et la fermeture de l'anneau autour de l'estomac pour réaliser une boucle fermée entre les deux extrémités 1 et 2, formant ainsi respectivement une première et une deuxième extrémités.

L'anneau gastrique conforme à l'invention se présente sous la forme d'un tore de révolution, de section par exemple sensiblement cylindrique, délimité extérieurement par une enveloppe 3 monocouche ou multicouche qui peut être avantageusement formée d'un revêtement de protection, par exemple à base de ou en silicone.

Tel qu'illustré en particulier aux figures 1 et 3, l'anneau gastrique conforme à l'invention comporte avantageusement un élément souple filiforme 4 présentant une bonne flexibilité et une bonne résistance mécanique, inséré longitudinalement et à coulissement selon l'axe principal de symétrie du cylindre, ou du corps principal de l'anneau, ledit élément 4 occupant la cavité reliant les première et deuxième extrémités 1,2 et s'étendant sensiblement entre les première et deuxième extrémités 1, 2, c'est-à-dire sensiblement sur toute la longueur développée de l'anneau.

Tel qu'illustré aux figures, l'élément souple filiforme 4 est monté pour définir ainsi une portion fixe 5 qui est solidarisée à l'aide de moyens de solidarisation 6, faisant par exemple intervenir un circlips et une rondelle, ou tout moyen équivalent, avec la première extrémité 1 de l'anneau. L'autre portion terminale de l'élément souple filiforme 4 forme une portion libre 7, c'est-à-dire susceptible de se déplacer par translation relativement à la portion fixe 5, ladite portion libre 7 étant associée fonctionnellement à un actionneur 8 monté sur l'anneau même vers ou au niveau de la deuxième extrémité 2. L'actionneur 8 est chargé de transmettre l'énergie nécessaire pour assurer, lorsqu'il est activé, la translation réversible de l'élément souple filiforme 4 à l'intérieur de l'anneau, i.e. le déplacement réversible de la portion libre 7 relativement à la portion fixe 5, en vue d'obtenir une variation associée du périmètre de l'anneau, c'est-à-dire une augmentation ou une réduction de son diamètre.

Le montage direct de l'actionneur 8 sur l'une des extrémités 2 de l'anneau permet ainsi un gain de place important et une bonne efficacité mécanique.

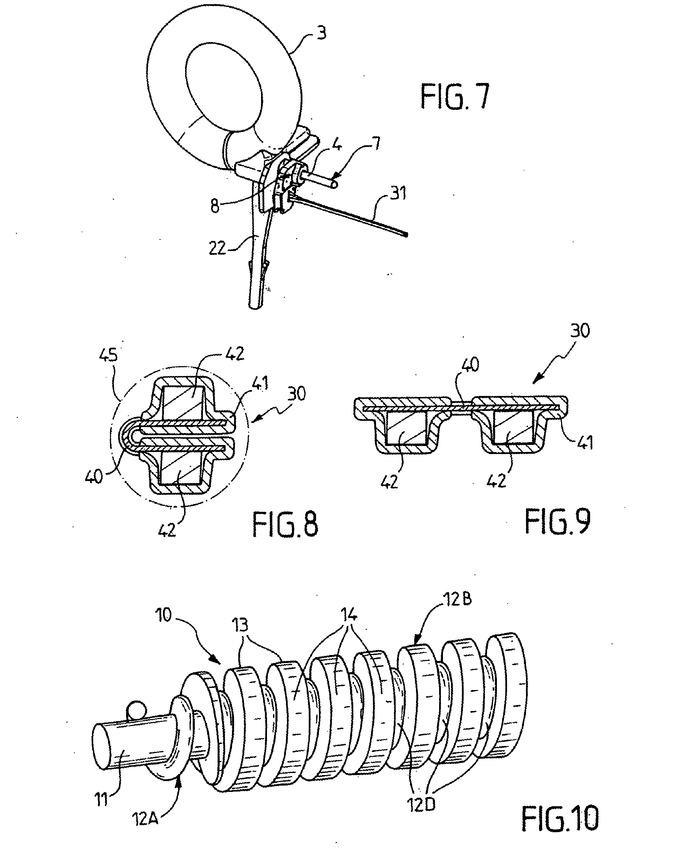

Avantageusement, la portion libre 7, s'étendant par exemple sur une longueur de l'ordre de quelques centimètres ou sur toute la longueur de l'élément souple filiforme, est pourvue de moyens de coopération de force 10 (figure 10) avec l'actionneur 8, ledit moyen 10 étant destiné à assurer la transmission de l'énergie fournie par l'actionneur 8 à l'ensemble de l'élément souple filiforme 4 à partir de son point d'appui matérialisé par la portion fixe 5.

Avantageusement, et tel qu'illustré à la figure 10, les moyens de coopération de force 10 sont formés par un pas de vis.

Selon l'invention, l'élément souple filiforme 4 présente une flexibilité suffisante pour pouvoir s'adapter à la forme sensiblement circulaire de l'anneau, tout en étant capable de transmettre la force nécessaire au réglage du diamètre de l'anneau. Avantageusement, l'élément souple filiforme 4 est formé par une âme souple 11, de préférence métallique, par exemple de section circulaire, sur laquelle est fixé et enroulé coaxialement, par exemple sur toute sa longueur, au moins un ressort à spires non jointives formant le pas de vis.

De manière particulièrement avantageuse, l'élément souple filiforme 4 comporte deux ressorts à spires non jointives pour former le pas de vis, respectivement un premier ressort 12A enroulé hélicoïdalement le long de l'âme souple 11 et un second ressort 12B de diamètre extérieur supérieur tel qu'illustré à la figure 10, et comportant préférentiellement des spires 14 de section transversale rectangulaire 13, de manière à délimiter une génératrice externe plane, ledit premier ressort 12A étant interposé entre les spires 14 du second ressort 12B pour maintenir un pas de vis carré constant.

Grâce à cette disposition, il est ainsi possible de conserver constamment un pas sensiblement constant et efficace, même en cas de déformation de l'élément souple filiforme 4. Ceci confère au dispositif une grande précision et une grande efficacité, tout en étant économe en énergie nécessaire à son fonctionnement en raison du rendement élevé de la transmission par une vis à pas carré.

Grâce à cette disposition, il est possible de garantir une position de réglage stable même lorsque aucune énergie n'est fournie au système.

Le second ressort 12B peut être avantageusement obtenu par découpage laser d'un tube cylindrique creux, son montage sur et entre les spires 12D du premier ressort 12A étant effectué après une traction longitudinale. Le second ressort 12B est donc animé naturellement d'une force élastique intrinsèque de compression tendant à rendre les spires jointives, cette force intrinsèque étant contrecarrée par les spires 12D du premier ressort 12A, contre lesquelles elles viennent en appui. On bénéficie ainsi d'un pas constant malgré l'élasticité et la flexibilité naturelles et indispensables de l'élément souple allongé 4.

L'actionneur 8 pourra être de tout moyen classique bien connu de l'homme du métier susceptible de coopérer avec le pas de vis pour lui transmettre un mouvement. De manière particulièrement avantageuse, l'actionneur 8 pourra être pourvu d'un simple écrou permettant d'assurer l'entraînement du pas de vis, l'actionneur 8 pouvant être de manière générale un moyen moteur, du genre moteur électrique, électromagnétique ou autre, sans pour autant sortir du cadre de l'invention.

A titre de variante de réalisation non représentée aux figures, il est bien évidemment possible de remplacer le pas de vis décrit ci-dessus par tout moyen technique équivalent, et par exemple par une crémaillère engrenant sur un actionneur 8 pourvu d'une roue dentée ou d'un moyen équivalent. On peut également envisager de réaliser l'élément souple filiforme 4 sous la forme d'un simple câble, entraîné de manière réversible par un actionneur 8 intégrant une poulie.

Tel qu'illustré aux figures, l'anneau gastrique conforme à l'invention est, de manière générale, formé par un corps principal à base d'un matériau compressible 20 formant le matériau de base et qui vient remplir l'intérieur de l'enveloppe 3. Dans le matériau compressible 20 est inséré longitudinalement et sensiblement, avec possibilité de coulissement, l'élément souple filiforme 4, tel qu'illustré par exemple aux figures 2 et 4.

De manière particulièrement avantageuse, le matériau compressible 20 est de l'ePTFE dont les caractéristiques de compressibilité et de stabilité à la striction conviennent particulièrement bien à ce genre d'application.

Selon une première variante de réalisation illustrée aux figures 1 et 2, l'anneau conforme à l'invention comprend une enveloppe 3 en matériau silicone et d'épaisseur sensiblement constante qui forme le revêtement extérieur étanche de l'anneau, l'intérieur de l'anneau étant formé exclusivement du matériau compressible 20, par exemple de l'ePTFE, à l'intérieur duquel l'élément souple filiforme 4 est inséré avec un léger jeu.

L'extrémité 1 comporte une poche 21, par exemple remplie de colle, et dans laquelle est montée et fixée, la portion fixe 5, avec les moyens de solidarisation 6.

Telle qu'illustrée aux figures 1 et 2, l'extrémité 1 est pourvue d'une languette 22 s'étendant vers l'extérieur de l'anneau et destinée à coopérer avec un élément complémentaire femelle 23, solidaire de la même extrémité 1 de l'anneau, pour constituer les moyens de fermeture 9, conduisant au verrouillage de l'anneau en formant, par exemple, une bride autour de l'extrémité 2.

Selon cette première variante de réalisation, l'action de l'actionneur 8 sur l'élément souple filiforme 4, transmet une force d'actionnement selon l'une des directions indiquées par la flèche F illustrée à la figure 1, ce qui a pour conséquence de comprimer ou relâcher, d'une manière sensiblement longitudinale, le matériau compressible 20 se traduisant par une variation associée du diamètre de l'anneau, tant interne qu'externe, sensiblement à la manière d'un noeud coulant.

La seconde variante de réalisation illustrée aux figures 3 et 4 ne diffère de celle illustrée aux figures 1 et 2 que par l'agencement spécifique de l'enveloppe externe 3 dont la périphérie dorsale 25 est renforcée en vue de brider l'extension radiale externe ou centrifuge de l'anneau pour, au contraire, privilégier la variation radiale interne ou centripète du diamètre de l'anneau. De cette manière, on privilégie la variation radiale du diamètre de l'anneau à sa périphérie interne qui est opposée à sa périphérie dorsale.

Telle qu'illustrée, la périphérie dorsale renforcée 25 peut être réalisée sous la forme d'une enveloppe externe 3 dont seule la périphérie dorsale présente une surépaisseur, c'est-à-dire une épaisseur dorsale externe plus importante que le reste de l'enveloppe externe (figure 4). Alternativement ou de manière complémentaire, la périphérie dorsale 25 peut être également réalisée en utilisant un matériau polymère de dureté supérieure à la dureté du reste de l'enveloppe en matériau polymère 3. Il est également envisageable, tel qu'illustré à la figure 4, de prévoir d'intégrer dans la périphérie dorsale renforcée 25 un insert de renfort 26, de préférence métallique s'étendant sur la majorité de la périphérie de l'anneau entre le matériau compressible 20 et la périphérie dorsale 25. Avantageusement, l'insert 26 peut présenter une mémoire de forme sensiblement circulaire pour obtenir une position de repos élastique circulaire de l'anneau.

Grâce à cet agencement, l'augmentation ou la réduction du diamètre de l'anneau est limitée à un déplacement radiale réversible, situé au niveau de la périphérie interne de l'anneau opposé à la périphérie dorsale, se traduisant par une variation du diamètre interne de l'anneau en direction centrifuge ou centripète, selon le sens de la sollicitation imprimée à l'élément souple filiforme 4, matérialisé par l'une des directions de la flèche F.

L'anneau gastrique conforme à l'invention est particulièrement conçu pour être intégré dans un système de restriction et de contrôle à distance de l'ingestion d'aliments dans l'estomac d'un patient, de telle manière que l'on puisse commander à distance, sans aucune intervention chirurgicale invasive, la variation du diamètre de l'anneau. A cette fin, l'actionneur 8 est un moteur électrique qui est avantageusement relié à un circuit de réception sous-cutané pourvu d'une antenne de réception 30 (figures 5 à 7) pour recevoir un signal radiofréquence de commande et de puissance, l'ensemble étant destiné à être implanté dans le corps du patient.

Tel qu'illustré notamment aux figures 5 à 7, le moteur électrique est solidarisé avec l'extrémité 2, de manière à être déporté à l'extérieur de l'anneau, le moteur électrique étant pourvu de manière classique d'un ensemble de paliers et engrenages, reliés fonctionnellement par une connexion électrique 31 au circuit de l'antenne de réception 30.

Dans cette application préférentielle, le moteur électrique est dépourvu de toute source d'alimentation interne, puisque son énergie est fournie par le circuit de réception 30 lequel convertit les ondes radio-fréquences reçues de l'unité de commande à travers l'antenne extérieure en signal de commande du moteur et en énergie pour assurer son alimentation électrique. L'antenne de réception 30 est adaptée et choisie pour recevoir à la fois un signal de commande et un signal de puissance.

Le faible besoin en énergie du moteur électrique permet d'envoyer par radiofréquence les ordres de commande et l'énergie d'actionnement du moteur évitant ainsi l'obligation d'avoir à implanter dans le corps même du patient une source additionnelle d'énergie telle qu'une pile ou une batterie.

Tel qu'illustré à la figure 5, le moteur électrique est relié à l'antenne de réception 30 par une connexion électrique 31 qui est protégée par une gaine 33 de protection assurant l'étanchéité et au bout de laquelle est montée ledit circuit de réception comportant l'antenne de réception 30. La portion libre 7 de l'élément souple filiforme 4 est également intégrée dans la gaine 33 de manière à obtenir un ensemble parfaitement protégé, étanche et susceptible d'agresser le moins possible les tissus environnants.

De manière particulièrement avantageuse, ledit circuit de l'antenne de réception 30 est repliable (figures 8 et 9) de manière élastique afin que le chirurgien puisse momentanément réduire les dimensions de la partie implantable du système c'est-à-dire l'anneau, la gaine 33 et le circuit de l'antenne de réception 30 pour faire passer l'ensemble monobloc dans un trocart de dimension faible, de préférence d'un diamètre inférieur par exemple à 15 mm, ceci afin de faciliter l'implantation.

Tel qu'illustré aux figures 8 et 9, le circuit de l'antenne de réception 30 repliable sera avantageusement mais non nécessairement flexible, soit en totalité, soit au moins en partie, et formée par un circuit électrique souple 40, se présentant par exemple sous la forme d'un disque enrobé dans une enveloppe silicone 41, cette dernière servant également de protection à des composant électroniques 42 connectés et reliés fonctionnellement à l'antenne proprement dite du circuit souple 40.

Selon une version particulièrement avantageuse de l'invention, le circuit de l'antenne de réception 30 se présentera sous la forme d'une pièce, par exemple en forme de disque repliable sur lui-même sensiblement selon le diamètre du disque tel que montré aux figures 5 et 6.

Grâce à cette disposition et aux propriétés d'élasticité et de souplesse des matériaux choisis, il est ainsi possible à partir de la position dépliée du circuit de l'antenne 30, telle qu'illustrée aux figures 6 et 9, de replier le circuit de l'antenne 30 le long de son diamètre pour occuper un volume restreint (figure 8), permettant son insertion dans un trocart de section circulaire, dont le contour 45 est représenté en ligne pointillée à la figure 8.

Le système de restriction et de contrôle à distance conforme à l'invention comporte également une antenne émettrice (non représentée aux figures) disposée à l'extérieur du patient pour envoyer un signal de commande et de puissance à l'antenne de réception 30, ladite antenne émettrice étant elle-même reliée fonctionnellement à une interface de commande, tel qu'un PC ou tout autre moyen équivalent à la disposition du médecin traitant.

En utilisation et une fois l'anneau gastrique conforme à l'invention implanté avec son circuit de réception muni de l'antenne 30 en position dépliée dans le corps du patient, le médecin traitant peut positionner sur la peau du patient l'antenne émettrice en position de face à face avec l'antenne de réception 30. Le médecin peut alors envoyer un signal de commande et de puissance en direction de l'antenne de réception 30, pour lui transmettre à la fois l'énergie nécessaire pour actionner l'actionneur 8 et en même temps commander son sens de déplacement.

Grâce au système de restriction et de contrôle à distance conforme à l'invention, il est ainsi possible de faire varier le diamètre de l'anneau gastrique sans avoir à pratiquer une intervention chirurgicale invasive et ce à volonté, puisque plusieurs cycles de commande peuvent être réalisés à intervalles réguliers ou non, sous la seule dépendance du médecin traitant.

Le système se révèle par ailleurs particulièrement sûr, puisque seul le médecin traitant possède le boîtier de commande comprenant l'antenne émettrice, ce qui lui permet d'exercer un contrôle total sur l'opération de réglage du diamètre. Le patient ne peut donc avoir librement accès à un moyen quelconque de réglage du diamètre de l'anneau.

L'invention concerne donc également un nouveau procédé de traitement chirurgical et thérapeutique mettant en oeuvre le système de restriction et de contrôle à distance de l'ingestion d'aliments conforme à l'invention.

## Revendications

1. - Anneau chirurgical destiné à être implanté dans le corps d'un patient autour d'organes biologiques constituant une poche ou un conduit pour d'une part former une boucle fermée entre ses deux extrémités (1,2), formant ainsi une première (1) et une deuxième (2) extrémités et, d'autre part, réduire le diamètre de l'ouverture dudit organe lorsqu'il est enserré par l'anneau, ledit anneau comprenant un système pour commander de manière réversible la variation de son diamètre, ledit système comportant un élément souple filiforme (4) inséré longitudinalement et à coulissement dans le matériau formant le corps de l'anneau sensiblement entre les première (1) et deuxième (2) extrémités, pour définir une portion fixe (5) solidarisée avec la première extrémité (1) et une portion libre (7) associée fonctionnellement à un actionneur (8) monté sur l'anneau vers la deuxième extrémité (2), de telle manière que l'actionneur (8) puisse assurer la translation réversible de l'élément souple filiforme (4) pour obtenir une variation associée du diamètre de l'anneau, la portion libre étant pourvue de moyens de coopération de force (10) avec l'actionneur (8), **caractérisé en ce que** les moyens de coopération de force (10) sont formés par un pas de vis, et l'élément souple filiforme (4) est formé par une âme souple (11) sur laquelle est fixé et enroulé coaxialement au moins un ressort à spires non jointives (12) formant le pas de vis.

2. Anneau selon la revendication 1 **caractérisé en ce que** l'élément souple filiforme (4) comporte deux ressorts à spires non jointives (12A, 12B) pour former le pas de vis, respectivement un premier ressort (12A) enroulé hélicoïdalement le long de l'âme souple (11) et un second ressort (12B) de diamètre extérieur supérieur et à spires (14) de section transversale rectangulaire, ledit premier ressort (12A) étant interposé entre les spires (14) du second ressort (12B) pour maintenir un pas de vis constant et à pas carré.

3. Anneau selon la revendication 1 ou 2 **caractérisé en ce que** l'actionneur (8) est pourvu d'un écrou pour assurer l'entraînement du pas de vis.

4. Anneau selon l'une des revendications 1 à 3 **caractérisé en ce qu'**il est formé par un corps principal à base d'un matériau compressible (20) dans lequel est inséré, avec possibilité de coulissement, l'élément souple filiforme (4).

5. Anneau selon la revendication 4 **caractérisé en ce que** le matériau compressible (20) est de l'ePTFE.

6. Anneau selon la revendication 4 ou 5 **caractérisé en ce qu'**il est pourvu extérieurement d'un revêtement de protection (3), par exemple de silicone.

7. Anneau selon l'une des revendications 1 à 6 **caractérisé en ce que** l'actionneur (8) est un moteur électrique.

8. Anneau selon la revendication 7 **caractérisé en ce que** le moteur électrique est relié à une antenne de réception (30) destinée à être implantée dans le corps du patient.

9. Anneau selon la revendication 7 ou 8 **caractérisé en ce que** l'antenne de réception (30) est repliable.

10. Anneau selon l'une des revendications 7 à 9 **caractérisé en ce que** le moteur électrique est relié à l'antenne de réception (30) par une connexion électrique (31) protégée par une gaine (33), au bout de laquelle est montée l'antenne de réception (30).

11. Anneau selon la revendication 9 ou 10 **caractérisé en ce que** l'antenne de réception (30) est formée par une pièce, par exemple en forme de disque, repliable sur lui-même sensiblement selon le diamètre du disque.

12. Anneau selon l'une des revendications 1 à 11 **caractérisé en ce que** son enveloppe externe (3) présente une périphérie dorsale renforcée (25), pour privilégier la variation radiale du diamètre de l'anneau à sa périphérie interne opposée à la périphérie dorsale.

13. Anneau selon la revendication 12 **caractérisé en ce que** la périphérie dorsale renforcée (25) est formée par une épaisseur dorsale de l'enveloppe externe plus importante que le reste de l'enveloppe externe (3) et/ou par un matériau polymère de dureté supérieure.

14. Anneau selon la revendication 12 ou 13 **caractérisé en ce que** la périphérie dorsale renforcée (25) comporte un insert de renfort (26), de préférence métallique.

15. Anneau selon l'une des revendications précédentes **caractérisé en ce qu'**il est formé par un anneau gastrique destiné à être implanté autour de l'estomac ou de l'oesophage.

16. Anneau selon l'une des revendications 1 à 14 **caractérisé en ce qu'**il est formé par un anneau destiné à être implanté autour de la vessie ou des voies urinaires ou autour des voies gastro-intestinales ou autour de vaisseaux sanguins.

17. Système de restriction et de contrôle à distance de l'ingestion d'aliments dans l'estomac d'un patient comportant :
- un anneau gastrique conforme à l'une des revendications 1 à 15 et comportant en tant qu'actionneur (8) un moteur électrique qui est relié à une antenne de réception (30) pour recevoir un signal de commande et de puissance,
- une antenne émettrice disposée à l'extérieur du patient pour envoyer un signal de commande et de puissance à l'antenne de réception (30), ladite antenne émettrice étant reliée fonctionnellement à une interface de commande.

## Claims

1. Chirurgical ring for being implanted into the body of a patient around biological organs constituting a pocket or a conduit for, on the one hand, forming a closed loop between its two extremities (1, 2), thereby forming a first (1) and a second (2) extremity and, on the other hand, reducing the diameter of the opening of said organ when it is constricted by the ring, said ring comprising a system for reversibly controlling the variation of the diameter, said system comprising an elastic filament-shaped element (4) which is longitudinally inserted and substantially sliding in the material forming the body of the ring between the first (1) and second (2) extremities, in order to define a fixed portion (5) solidarised with the first extremity (1) and a free portion (7) functionally associated to an operating gear (8) installed on the ring in the direction to the second extremity (2), so that the operating gear (8) allows the reversible translation of the elastic filament-shaped element (4) in order to obtain an associated variation of the ring diameter, the free portion being provided with drive means (10) cooperating with the operating gear (8), **characterized in that** the drive means (10) are formed by a screw, and the elastic filament-shaped element (4) is formed by an elastic core (11), wherein at least a winding with non-joined springs (12) forming the screw is fixed and coaxially wound-up on the elastic core.

2. Ring according to claim 1, **characterized in that** the elastic filament-shaped element (4) contains two windings with non-joined springs (12A, 12B) for forming the screw, i.e. a first winding (12A) which is helicoidally coiled-up along the elastic core (11) and a second winding (12B), respectively, having a greater outer diameter and having springs (14) of rectangular transversal section, the first winding (12A) being interposed between the springs (14) of the second winding (12B) in order to maintain a square screw with constant pitch.

3. Ring according to claim 1 or 2, **characterized in that** the operating gear (8) comprises a screw nut for driving the screw.

4. Ring according to any one of claims 1 to 3, **characterized in that** it is formed by a main body on the basis of a compressible material (20) in which the elastic filament-shaped element (4) is slidingly inserted.

5. Ring according to claim 4, **characterized in that** the compressible material (20) is PTFE.

6. Ring according to claim 4 or 5, **characterized by** an outer protection layer (3), for example constituted of silicone.

7. Ring according to any one of claims 1 to 6, **characterized in that** the operating gear (8) is an electric motor.

8. Ring according to claim 7, **characterized in that** the electric motor is connected to a receiving antenna (30) for being implanted into the body of a patient.

9. Ring according to claim 7 or 8, **characterized in that** the receiving antenna (30) is foldable.

10. Ring according to any one of claims 7 to 9, **characterized in that** the electric motor is connected to the receiving antenna (30) by an electric connection (31) which is protected by a sheath (33) at the end of which the receiving antenna (30) is arranged.

11. Ring according to claim 9 or 10, **characterized in that** the receiving antenna (30) is formed of one piece, for example in the form of a disc, foldable on itself essentially according to the diameter of the disc.

12. Ring according to any one of claims 1 to 11, **characterized in that** its outer envelope (3) presents a reinforced dorsal periphery (25) in order to facilitate the radial variation of the ring diameter to its inner periphery opposed to the dorsal periphery.

13. Ring according to claim 12, **characterized in that** the reinforced dorsal periphery (25) is formed by a greater dorsal thickness of the outer envelope than the rest of the outer envelope (3) and/or by a polymeric material of superior hardness.

14. Ring according to claim 12 or 13, **characterized in that** the reinforced dorsal periphery (25) comprises a reinforcing insert (26) preferably made of metal.

15. Ring according to any one of the preceding claims, **characterized in that** it is formed by a gastric ring for being implanted around the stomach or the esophagus.

16. Ring according to any one of claims 1 to 14, **characterized in that** it is formed by a ring for being implanted around the bladder or the urinary passages or around gastro-intestinal passages or around blood vessels.

17. Remote controlled restriction system for aliment ingestion into the stomach of a patient comprising:
- a gastric ring according to any one of claims 1 to 15 and containing an electric motor as an operating gear (8), said electric motor being connected to a receiving antenna (30) for receiving a control and power signal,
- an emitting antenna disposed outside the patient for sending a control and power signal to the receiving antenna (30), the emitting antenna being functionally connected to a control interface.

## Patentansprüche

1. Chirurgischer Ring zum Implantieren in den Körper eines Patienten um biologische Organe, die eine Tasche oder einen Kanal bilden, einerseits zum Bilden einer geschlossenen Schlaufe zwischen seinen beiden äußersten Enden (1, 2), wodurch ein erstes (1) und ein zweites (2) äußerstes Ende gebildet ist, und andererseits zum Reduzieren des Durchmessers der Öffnung des Organs, wenn es durch den Ring konstringiert wird, wobei der Ring ein System zum reversiblen Steuern der Änderung des Durchmessers aufweist, das System ein elastisches fadenförmiges Element (4) aufweist, das in dem den Körper des Rings bildenden Material zwischen dem ersten (1) und zweiten (2) äußersten Ende längs eingesetzt ist und im wesentlichen gleitet, um eine feste Position (5) zusammen mit dem ersten äußersten Ende (1) und einen freien Abschnitt (7) in funktioneller Zuordnung zu einem Betätigungsorgan (8) zu bilden, das am Ring in Richtung zum zweiten äußersten Ende (2) so angebaut ist, daß das Betätigungsorgan (8) die reversible Translation des elastischen fadenförmigen Elements (4) ermöglicht, um eine damit zusammenhängende Änderung des Ringdurchmessers zu erhalten, und der freie Abschnitt mit einer Antriebseinrichtung (10) versehen ist, die mit dem Betätigungsorgan (8) zusammenwirkt, **dadurch gekennzeichnet, daß** die Antriebseinrichtung (10) durch eine Spindel gebildet ist und das elastische fadenförmige Element (4) durch einen elastischen Kern (11) gebildet ist, wobei mindestens eine Windung mit nicht verbundenen Federn (12), die die Spindel bilden, auf dem elastischen Kern befestigt und darauf koaxial gewunden ist.

2. Ring nach Anspruch 1, **dadurch gekennzeichnet, daß** das elastische fadenförmige Element (4) zwei Windungen mit nicht verbundenen Federn (12A, 12B) zum Bilden der Spindel enthält, d. h., eine erste Windung (12A), die entlang dem elastischen Kern (11) schraubenförmig gewunden ist, bzw. eine zweite Windung (12B) mit einem größeren Außendurchmesser und mit Federn (14) mit rechtwinkligem Querschnitt, wobei die erste Windung (12A) zwischen den Federn (14) der zweiten Windung (12B) eingefügt ist, um eine Flachgewindespindel mit konstanter Steigung beizubehalten.

3. Ring nach Anspruch 1 oder 2, **dadurch gekennzeichnet, daß** das Betätigungsorgan (8) eine Spindelmutter zum Antreiben der Spindel aufweist.

4. Ring nach einem der Ansprüche 1 bis 3, **dadurch gekennzeichnet, daß** er durch einen Hauptkörper auf der Grundlage eines zusammendrückbaren Materials (20) gebildet ist, in dem das elastische fadenförmige Element (4) gleitend eingesetzt ist.

5. Ring nach Anspruch 4, **dadurch gekennzeichnet, daß** das zusammendrückbare Material (20) PTFE ist.

6. Ring nach Anspruch 4 oder 5, **gekennzeichnet durch** eine Außenschutzschicht (3), die z. B. aus Silikon gebildet ist.

7. Ring nach einem der Ansprüche 1 bis 6, **dadurch gekennzeichnet, daß** das Betätigungsorgan (8) ein Elektromotor ist.

8. Ring nach Anspruch 7, **dadurch gekennzeichnet, daß** der Elektromotor mit einer Empfangsantenne (30) zum Implantieren in den Körper eines Patienten verbunden ist.

9. Ring nach Anspruch 7 oder 8, **dadurch gekennzeichnet, daß** die Empfangsantenne (30) klappbar ist.

10. Ring nach einem der Ansprüche 7 bis 9, **dadurch gekennzeichnet, daß** der Elektromotor mit der Empfangsantenne (30) durch eine elektrische Verbindung (31) verbunden ist, die durch einen Mantel (33) geschützt ist, an dessen Ende die Empfangsantenne (30) angeordnet ist.

11. Ring nach Anspruch 9 oder 10, **dadurch gekennzeichnet, daß** die Empfangsantenne (30) aus einem Stück ausgebildet ist, z. B. in Form einer Scheibe, die im wesentlichen gemäß dem Durchmesser der Scheibe zusammenklappbar ist.

12. Ring nach einem der Ansprüche 1 bis 11, **dadurch gekennzeichnet, daß** seine Außenhülle (3) eine verstärkte dorsale Peripherie (25) darstellt, um die Radialänderung des Ringdurchmessers zu seiner Innenperipherie entgegengesetzt zur dorsalen Peripherie zu erleichtern.

13. Ring nach Anspruch 12, **dadurch gekennzeichnet, daß** die verstärkte dorsale Peripherie (25) durch eine größere dorsale Dicke der Außenhülle als der Rest der Außenhülle (3) und/oder durch ein Polymermaterial mit größerer Härte gebildet ist.

14. Ring nach Anspruch 12 oder 13, **dadurch gekennzeichnet, daß** die verstärkte dorsale Peripherie (25) einen vorzugsweise aus Metall hergestellten Verstärkungseinsatz (26) aufweist.

15. Ring nach einem der vorstehenden Ansprüche, **dadurch gekennzeichnet, daß** er durch einen Magenring zum Implantieren um den Magen oder die Speiseröhre gebildet ist.

16. Ring nach einem der Ansprüche 1 bis 14, **dadurch gekennzeichnet, daß** er durch einen Ring zum Implantieren um die Blase oder die Harntrakte oder um Margen-Darm-Trakte oder um Blutgefäße gebildet ist.

17. Ferngesteuertes Restriktionssystem für die Nahrungsaufnahme in den Magen eines Patienten mit:
- einem Magenring nach einem der Ansprüche 1 bis 15, der einen Elektromotor als Betätigungsorgan (8) enthält, wobei der Elektromotor mit einer Empfangsantenne (30) zum Empfangen eines Steuer- und Leistungssignals verbunden ist,
- einer außerhalb des Patienten angeordneten Sendeantenne zum Senden eines Steuer- und Leistungssignals zur Empfangsantenne (3.0), wobei die Sendeantenne mit einer Steuerschnittstelle funktionell verbunden ist.
